# EUROPÄISCHE PATENTSCHRIFT

(11) **EP 1 504 818 B1**
(45) Veröffentlichungstag und Bekanntmachung des Hinweises auf die Patenterteilung: **16.04.2008**
(21) Anmeldenummer: 04018862.5
(22) Anmeldetag: 09.08.2004
(51) Int. Cl.: B01L 3/00, G01N 1/10, G01N 27/28, G01N 33/18

(54) **Durchflussgefäss zur Wasseranalytik**
Flow-through container for analysing water
Contenant à écoulement traversant pour analyse d'eau

(30) Priorität: 07.08.2003 DE 10336912
(43) Veröffentlichungstag der Anmeldung: 09.02.2005
(73) Patentinhaber: Schierjott, Günter, Dr. techn., D-44791 Bochum (DE)
(72) Erfinder: Schierjott, Günter, Dr. techn., D-44791 Bochum (DE)
(74) Vertreter: Thiel, Christian

(56) Entgegenhaltungen:
- DE-A- 2 830 313
- DE-A- 10 016 023
- US-A- 5 120 129
- US-A- 5 945 830

## Beschreibung

Die Erfindung betrifft ein Messgefäß, bestehend aus einem Rohr mit Zu- und Ableitung für ein zu messendes Fluid, einer Aufnahme für eine Messsonde an einem Rohrende sowie einem Abschluß am anderen Rohrende, das insbesondere für die Wasseranalytik geeignet und bestimmt ist.

Herkömmliche Durchflußgefäße sind insbesondere in der Wasseranalytik vielfach im Einsatz. Ein weiteres Einsatzgebiet ist die Lebensmittel- und Getränkeindustrie. Es handelt sich dabei um Rohre, die mit je einem Zu- und Abfluß für das zu messende Wasser versehen sind sowie eine Aufnahme aufweisen, in die die Meßsonde eingeführt werden kann.

Als Meßsonden kommen insbesondere pH-Einstabelektroden, Leitfähigkeitsaufnehmer, aber auch Meßsonden für bestimmte Inhaltsstoffe des zu messenden Wassers in Frage, beispielsweise Gassonden und insbesondere Sauerstoffsonden. Die Meßsonden sind über ein Außengewinde in ein entsprechendes Innengewinde der Aufnahme dichtend eingepaßt. Bei der Aufnahme selbst handelt es sich um eine Art durchbrochenen Stopfen, der in das Durchflußgefäß stirnseitig eingeschraubt oder eingeschweißt ist.

Herkömmliche Durchflußgefäße besitzen ein relativ großes Volumen von u. U. mehr als 100 ml. Bei geringer Durchflußmenge bedeutet dies, daß sprunghafte Änderungen des zu messenden Parameters nicht immer so zeitnah erfaßt werden können, wie dies wünschenswert wäre. Der Zeitpunkt eines Sprungs verschiebt sich auf der Zeitachse nach hinten und die Veränderung stellt sich weniger steil dar als sie tatsächlich auftritt. In der Wasseranalytik ist aber die zeitnahe Reaktion auf sich ändernde Parameter oft wichtig.

Des weiteren ist die Fertigung von Durchflußgefäßen vom rohrförmigen Typ mit an den beiden Enden eingeschweißten oder eingeschraubten Verschluß- und Anschlußelementen kostenaufwendig. Die aufwendige Bauweise steht auch der Miniaturisierung solcher Durchflußgefäße entgegen.

Ein Meßgefäß, das den Stand der Technik entspricht ist aus dem Dokument US-A-5 945 830 bekannt.

Aufgabe ist deshalb die Bereitstellung eines Meßgefäßes, das einfach und kostengünstig herzustellen ist und gleichzeitig im Meßraum ein reduziertes Volumen aufweist.

Diese Aufgabe wird mit einem Meßgefäß der Eingangs beschriebenen Art gelöst, bei der zumindest die Aufnahme für die Messsonde und gegebenenfalls der Abschluß des Rohres in die Rohrenden eingeklebt und/oder eingequetscht sind. Die erfindungsgemäßen Meßgefäße sind vorzugsweise Druckgefäße.

Erfindungsgemäß werden die Abschlüsse des Gefäßes, nämlich die Aufnahme für die Messsonde und der Abschluß am Rohrende (der gegebenenfalls auch eine Messsonde aufnehmen kann) formschlüssig zu den Rohrenden hergestellt und mit einem kommerziellen druckfesten Klebstoff dichtend in die Rohrenden eingeklebt. Derartige Klebstoffe, die auch in temperaturbeständigen und korrosionsbeständigen Ausführungen verfügbar sind und mit denen Metalle dauerhaft verbunden werden können, sind in der Technik an und für sich bekannt und verbreitet. Sie haben sich auch für den genannten Zweck als geeignet erwiesen.

Der Abschluß des Rohres ist vorzugsweise, ebenso wie die Aufnahme, in das Rohrende eingeklebt. Alternativ kann der Abschluß auch als eingequetschter bzw. eingeschrumpfter bzw. O-Ring-gedichteter Stopfen vorliegen. Auch Kombinationen vom Kleb- und Quetschverbindungen sind möglich.

Die erfindungsgemäßen Meßgefäße sind, was sowohl das Rohr, die Anschlüsse, die Aufnahme und den Abschluß anbetrifft, vorzugsweise aus Metall und insbesondere aus einem dafür üblichen Edelstahl. Das Rohr selbst kann an seinen Enden, im Bereich des Sitzes der Aufnahme und des Abschlusses, aufgeweitet sein, kann jedoch auch insgesamt geradlinig verlaufen. Zu- und Ablauf für das zu messende Fluid befinden sich an entgegengesetzten Enden des Rohres, auch der Zulauf vorzugsweise in seitlicher Stellung. Der Zulauf kann aber auch im Bereich des Abschlusses angeordnet sein.

Die Aufnahme selbst hat die Form einer Hülse, deren Außendurchmesser dem Innendurchmesser des Rohres am Einsatzort entspricht. Es ist sinnvoll, ein gewisses Spiel für die einzubringende Klebstoffschicht vorzusehen.

Die Aufnahme oder Hülse selbst weist eine konzentrische Durchbrechung oder Bohrung auf, die zur Aufnahme einer Meßsonde bestimmt ist. Die Durchbrechung oder Bohrung hat die für solche Aufnahmen üblichen Abmessungen und weist in der Regel im oberen Bereich ein Gewinde auf, in der ein entsprechendes Außengewinde der Sondeneinrichtung paßt. Ein in der Bohrung vorgesehener konischer Abschnitt wirkt mit einem an der Sondeneinrichtung vorhandenen Dichtmittel, etwa einer Manschette oder einem O-Ring, zusammen.

Die Sondeneinrichtung bzw. der Meßfühler kann auch in eine glattwandige zylindrische oder nach innen konisch zulaufende Bohrung eingequetscht sein. Eine Quetschverbindung kann beispielsweise mit Hilfe eines elastischen Elements, etwa einer Gummimanschette hergestellt werden. Eine Einbringung über eine Klemmring/Schneidringverschraubung ist ebenfalls möglich. Bei einer Gummimanschette wird die Elastizität des Gummis zur Herstellung einer hochdruckfesten Verbindung genutzt, bei Verwendung eines Metallelements die Plastizität des Metalls, vorzugsweise Edelstahls.

In jedem Fall ist die Messsonde bzw. der Meßfühler in der Aufnahme so festgelegt, daß sie, bzw. er zu Wartungszwecken bzw. zum Austausch wieder entnommen werden kann.

Bei der Messsonde handelt es sich um eine übliche Sonde, wie sie zum direkten Einbau in Prozessleitungen oder -behältern oder in Durchflussgefäßen verwandt und von zahlreichen Firmen bereitgestellt werden. Besonders geeignet sind die erfindungsgemäßen Meßgefäße für pH-Einstabelektroden, Leitfähigkeitsaufnehmer und Sauerstoffsonden zur Bestimmung des gelösten Sauerstoffs. Weiterhin können aber auch Temperaturfühler eingesetzt werden sowie Spezialsonden zur Feststellung von Inhaltsstoffen in dem zu messenden Fluid. Als Fluide werden Gase, Flüssigkeiten und Mischungen derselben angesehen. Gemessen werden Flüssigkeiten aller Art, wäßrig oder nicht-wäßrig, oder auch Gase und Mischungen von Flüssigkeiten und Gasen. Bei entsprechender Druck- und Temperaturbeständigkeit der eingesetzten Messsonde können die erfindungsgemäßen Messgefäße bei Drucken bis zu 60bar und Temperaturen bis zu 160°C eingesetzt werden. Bei Verwendung des Gefäßes zu Gasmessungen mit Sensoren für brennbare Gase, Sauerstoffsensoren, Sensoren für korrosive oder toxische Gase, etc. sind die Anschlüsse vorzugsweise gasdicht ausgelegt.

Am der Aufnahme gegenüberliegenden Ende des Meßgefäßes ist dieses mit einem Abschluß versehen, in der Regel einem Stopfen, der auf die gleiche Art und Weise in das Rohrende eingepaßt und dort mit Hilfe des Klebstoffs und/oder der Quetschverbindung festgelegt ist. Der Abschluß selbst kann auch die Zuleitung aufnehmen oder als Halterung für eine weitere Meßsonde oder als reiner Stopfen dienen. In letzterem Fall führt die Zuleitung für das zu messende Fluid seitlich in das Gefäß hinein.

In einer bevorzugten Ausführungsform dient der Abschluß als Aufnahme oder Halterung für ein weiteres funktionales Element, beispielsweise eine Strahlungsquelle, die mit einem auf der Aufnahmeseite eingebrachten Meßfühler für beispielsweise Extinktions- oder Turbiditätsmessungen zusammenwirkt.

Die Verwendung des unteren Abschlusses als Halterung für eine Messsonde ist auch dann sinnvoll, wenn es um die Messung von Kondensaten mit größeren Gasanteilen oder von Gas-Wasser-Gemischen geht, in denen beispielsweise der pH-Wert oder Leitfähigkeit der flüssigen Phase bestimmt werden soll. Bei geeigneter Lage der Zu- und Ableitungen kann entsprechend das erfindungsgemäße Meßgefäß als Trenngefäß verwandt werden, das alternativ oder gleichzeitig, je nach Anordnung der Messsonden, die flüssige und/oder die gasförmige Phase messen kann. Bei der gleichzeitigen Messung der flüssigen und der gasförmigen Phase ist gegebenenfalls ein weiterer Anschluss für die Zuführung des zu messenden Fluids vorzusehen, der zwischen der oberen und unteren Ableitung angeordnet ist.

Schließlich ist die Ausbildung des Abschlusses als zusätzliche Zuleitung beispielsweise für die Zuleitung von Reinigungsmitteln, Prüfflüssigkeiten oder Kalibrierungsmedien geeignet.

Bei Ausbildung des Abschlusses als Stopfen weist dieser bevorzugt auch einen Erdungsanschluß auf. Der Erdungsanschluß kann in Form einer Erdungsschraube vorliegen, die in eine Blindbohrung mit Innengewinde in den Stopfen eingeführt wird. Die Blindbohrung mit Innengewinde kann beispielsweise auch zur Aufnahme eines Temperaturfühlers dienen, wobei dieser Temperaturfühler vorzugsweise in einer Metallhülse mit Außengewinde angeordnet ist. Auch in diesem Fall kann die Erdung über den Stopfen erfolgen, beispielsweise über eine Kontermutter im Bereich der Metallhülse des Temperaturfühlers. Der Erdungsanschluß kann zusätzlich den sonst üblichen Potentialstab vorteilhaft ersetzen.

Bei Betrieb des erfindungsgemäßen Meßgefäßes mit einer pH-Sonde ergibt sich ein weiterer Vorteil bei der Messung von ionenarmen Fluiden. Destilliertes oder deionisiertes Wasser oder organische Flüssigkeiten ergeben bei der pH-Bestimmung mit üblichen pH-Elektroden aufgrund der geringen Ionenkonzentration häufig nur ungenaue Meßwerte. Diese Ungenauigkeit wird bei herkömmlichen Durchflußgefäßen auch nicht dadurch behoben, daß die Elektroden durch ihr Diaphragma geringe Ionenmengen in die Umgebung abgeben; in dem relativ großen Volumen führt die dann immer noch geringe Ionenkonzentration nicht zu einer Verbesserung der Situation. Bei den erfindungsgemäßen Meßgefäßen, die mit einem Volumen im Gefäßbereich von 50 bis 20 ml und weniger auskommen, führt aber die Ionendiffusion aus der Elektrode heraus bereits zu einer nennenswerten Veränderung der Ionenkonzentration. Auf diese Art und Weise können in ionenarmen Wässern pH-Bestimmungen mit einer bislang nicht möglichen Genauigkeit durchgeführt werden.

Bei Nutzung des Meßgefäßes für Leitfähigkeitsmessungen kann die Ableitungsfunktion in herkömmlicher Weise über die Aufnahme erfolgen. Bevorzugt ist jedoch die Anordnung der Ableitungsfunktion im Bereich des Abschlusses, insbesondere im Bereich des Erdungsanschlusses. Im übrigen wird die Leitfähigkeitssonde auf konventionelle Art und Weise angeordnet und betrieben. Die Anordnung der Ableitungsfunktion im Bereich des Abschlusses ist technisch einfacher als die herkömmliche Anordnung im Bereich der Aufnahme für die Meßsonde.

Das erfindungsgemäße Meßgefäß erlaubt eine einfache Inspektion und Reinigung von oben durch die Aufnahme nach Entfernung der Meßsonde oder durch den entsprechend ausgebildeten Abschluß.

Das erfindungsgemäße Meßgefäß erlaubt die Messung von Gasen, Flüssigkeiten oder Gemischen derselben. Darüber hinaus ist es besonders geeignet, auch zur Messung von gelösten Gasen, beispielsweise des Sauerstoffgehaltes in Wasser mit Hilfe einer O₂-Sonde.

In der Regel sind die erfindungsgemäßen Meßgefäße für den Bypass-Betrieb mit extraktiver Probeentnahme vorgesehen. Die Anordnung kann aber, insbesondere dann, wenn es um Fluidströme mit geringem Volumen geht, auch mit dem Gesamtstrom des zu messenden Mediums durchströmt werden, d.h. die Messwerte werden im Fluid direkt gewonnen und die Meßwerte werden im Fluid direkt gewonnen und nach außen übertragen, ohne daß eine Probe genommen werden muß.

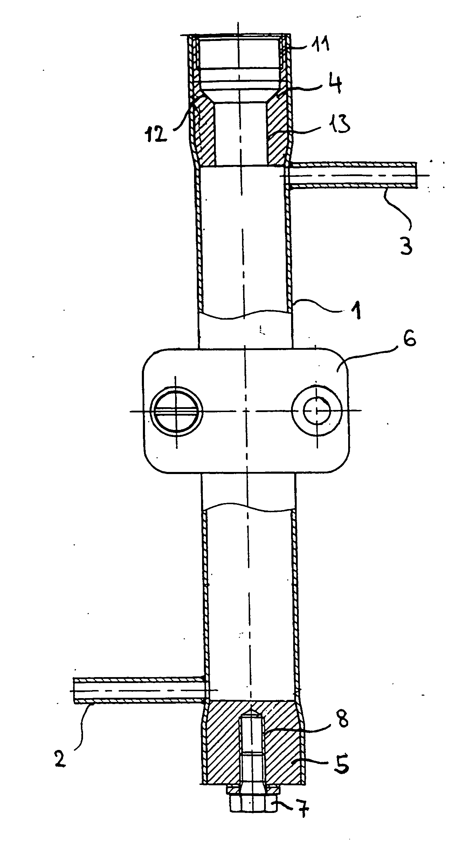

Die Erfindung wird durch die Abbildung näher erläutert.

Die Abbildung zeigt ein hochdruckfestes Meßgefäß mit einem Rohr 1, das an seinem unteren Ende eine Zuleitung 2 und an seinem oberen Ende eine Ableitung 3 für das zu messende Medium, in der Regel Wasser, aufweist. Zu- und Ableistung befinden sich unmittelbar angrenzend an die Hülse 4 bzw. den Stopfen 5 um Totvolumina zu vermeiden. Am oberen Ende befindet sich die Hülse 4, die in das leicht aufgeweitete Rohr 1 formschlüssig eingepaßt und eingeklebt ist. Am unteren Ende befindet sich der Stopfen 5, der das Rohr nach unten hin abschließt und eine Blindbohrung 8 aufweist, in deren Gewinde eine Erdungsschraube 7 eingeschraubt ist. Hier kann alternativ über eine Hülse ein Thermofühler angeordnet werden, der über eine Kontermutter am Abschluß gesichert wird, wobei die Kontermutter gleichzeitig als Erdungsanschluß dient.

Im zentralen Bereich des Rohres 1 befindet sich eine herkömmliche Befestigungsschelle 6 mit Bohrungen/Schrauben zur Festlegung an einer Oberfläche.

Die Aufnahme 4 selbst weist in ihrem oberen Bereich ein Gewinde 11 auf, in das die Meßsonde mit einem Außengewinde eingeschraubt wird. Im weiteren Verlauf findet sich ein konisch verlaufender Abschnitt 12, der als Dichtfläche für ein Dichtungselement an der Meßsonde dient. Im übrigen wird die Meßsonde durch den weiteren geraden Abschnitt 13 hindurch in das Meßgefäß geführt, wo sie ihre Meßfunktion erfüllt.

Für die Verbindung zwischen Aufnahme 4 bzw. Abschluß 5 einerseits und Rohr 1 andererseits können übliche Klebstoffe verwandt werden. Bei der Verklebung der Elemente muß berücksichtigt werden, ob eine leitende Verbindung mit der Wand des Rohres 1 benötigt wird. Ist dies der Fall, beispielsweise um Erdungs- und Ableitungsfunktionen übernehmen zu können, kann durch entsprechende Zusätze zum Klebstoff, beispielsweise in Form von Graphit- oder Metallpulver, eine leitende Verbindung hergestellt werden, oder durch einen entsprechenden Kontakt über klebstofffreie Stellen, beispielsweise im Bereich der Verjüngung der aufgeweiteten Stellen des Rohres 1 in den Endbereichen.

Vorzugsweise sind die erfindungsgemäßen Meßgefäße aus Edelstahl gefertigt. Es ist jedoch ohne weiteres möglich, andere Materialien zu verwenden, beispielsweise korrosionsfeste Sonderlegierungen, Kunststoffe für Niederdruckanwendungen, aber auch Glas zur Erleichterung der optischen Inspektion.

## Patentansprüche

1. Meßgefäß, insbesondere für die Wasseranalytik, bestehend aus einem Rohr (1) mit Zu- (2) und Ableitung (3) für ein zu messendes Fluid, wenigstens einer Aufnahme (4) für eine Messsonde an einem Rohrende sowie einem Abschluß (5) am anderen Rohrende, **dadurch gekennzeichnet, daß** zumindest die Aufnahme (4) und ggf. der Abschluß (5) in die Rohrenden eingeklebt und/oder eingequetscht sind.

2. Meßgefäß nach Anspruch 1, **dadurch gekennzeichnet, daß** Aufnahme (4) und/oder Abschluß (5) aus Metall sind.

3. Meßgefäß nach Anspruch 1 oder 2, **dadurch gekennzeichnet, daß** die Aufnahme (4) die Form einer Hülse mit zentraler Durchbrechung für den Meßfühler hat.

4. Meßgefäß nach Anspruch 3, **dadurch gekennzeichnet, daß** die Hülse ein Normgewinde aufweist.

5. Meßgefäß nach einem der vorstehenden Ansprüche, **dadurch gekennzeichnet, daß** der Abschluß (5) als Stopfen ausgebildet ist.

6. Meßgefäß nach Anspruch 5, **dadurch gekennzeichnet, daß** der Stopfen einen elektrischen Erdungsanschluß (7) aufweist.

7. Meßgefäß nach Anspruch 6, **dadurch gekennzeichnet, daß** der Erdungsanschluß (7) zusätzlich als Potentialableitung dient.

8. Meßgefäß nach Anspruch 5 bis 7, **dadurch gekennzeichnet, daß** der Stopfen (5) mit Bohrung (8) und Gewinde ausgestattet ist.

9. Meßgefäß nach einem der Ansprüche 5 bis 8, **dadurch gekennzeichnet, daß** im Stopfen (5) ein Temperaturfühler angeordnet ist.

10. Meßgefäß nach Anspruch 9, **dadurch gekennzeichnet, daß** der Temperaturfühler eine Metallhülse mit einem Außengewinde aufweist.

11. Meßgefäß nach einem der vorstehenden Ansprüche, **dadurch gekennzeichnet, daß** seine Elemente aus Edelstahl bestehen.

12. Meßgefäß nach einem der Ansprüche 1 bis 4, **dadurch gekennzeichnet, daß** der Abschluß (5) als Aufnahme (4) für ein Meßelement ausgebildet ist.

13. Meßgefäß nach einem der vorstehenden Ansprüche mit einer Druckfestigkeit von bis zu 2x10⁶ Pa (20 bar).

## Claims

1. A measuring vessel, particularly for water analysis, consisting of a pipe (1) with a supply line (2) and a discharge line (3) for a fluid to be measured, at least one receptacle for a measuring sensor at one end of the tube and a closure (5) at the other end of the tube, **characterised in that** at least the receptacle (4) and optionally the closure (5) are secured in the ends of the tube by adhesive and/or are compression fitted.

2. A measuring vessel as claimed in Claim 1, **characterised in that** the receptacle (4) and/or closure (5) are metallic.

3. A measuring vessel as claimed in Claim 1 or 2, **characterised in that** the receptacle (4) has the form of a sleeve with a central opening for the measuring sensor.

4. A measuring vessel as claimed in Claim 3, **characterised in that** the sleeve has a standard screw thread.

5. A measuring vessel as claimed in one of the preceding claims, **characterised in that** the closure (5) is in the form of a plug.

6. A measuring vessel as claimed in Claim 5, **characterised in that** the plug has an electrical earthing connection (7).

7. A measuring vessel as claimed in Claim 6, **characterised in that** the earthing connection (7) serves additionally as a potential grounding.

8. A measuring vessel as claimed in Claims 5 to 7, **characterised in that** the plug (5) is provided with a bore (8) and a screw thread.

9. A measuring vessel as claimed in one of Claims 5 to 8, **characterised in that** a temperature sensor is disposed in the plug (5).

10. A measuring vessel as claimed in Claim 9, **characterised in that** the temperature sensor includes a metal sleeve with an external screw thread.

11. A measuring vessel as claimed in one of the preceding claims, **characterised in that** its elements consist of high-quality steel.

12. A measuring vessel as claimed in one of Claims 1 to 4, **characterised in that** the closure (5) is constructed as a receptacle (4) for a measuring element.

13. A measuring vessel as claimed in one of the preceding claims with a pressure resistance of up to 2 x 10⁶ Pa (20 bar).

## Revendications

1. Récipient de mesure, en particulier pour l'analyse de l'eau, consistant en un tube (1) ayant une conduite d'entrée (2) et une conduite de sortie (3) pour un fluide à mesurer, au moins un logement (4) pour une sonde de mesure à une extrémité du tube, ainsi qu'une obturation (5) à l'autre extrémité du tube, **caractérisé en ce qu'**au moins le logement (4) et éventuellement l'obturation (5) sont collés et/ou sertis dans les extrémités de tube.

2. Récipient de mesure selon la revendication 1, **caractérisé en ce que** le logement (4) et/ou l'obturation (5) sont en métal.

3. Récipient de mesure selon la revendication 1 ou 2, **caractérisé en ce que** le logement (4) a la forme d'une gaine avec évidement central pour le capteur de mesure.

4. Récipient de mesure selon la revendication 3, **caractérisé en ce que** la gaine présente un filetage standard.

5. Récipient de mesure selon l'une quelconque des revendications précédentes, **caractérisé en ce que** l'obturation (5) à la configuration d'un bouchon.

6. Récipient de mesure selon la revendication 5, **caractérisé en ce que** le bouchon présente une mise à la terre élec trique (7).

7. Récipient de mesure selon la revendication 6, **caractérisé en ce que** la mise à la terre sert en outre de dérivation de potentiel.

8. Récipient de mesure selon la revendication 5 à 7, **caractérisé en ce que** le bouchon (5) est muni d'un trou taraudé (8).

9. Récipient de mesure selon l'une des revendications 5 à 8, **caractérisé en ce que** dans le bouchon (5) est placée une sonde de température.

10. Récipient de mesure selon la revendication 9, **caractérisé en ce que** la sonde de température présente une gaine en métal avec un filetage externe.

11. Récipient de mesure selon l'une des revendications précédentes, **caractérisé en ce que** ses éléments sont constitués d'acier spécial.

12. Récipient de mesure selon l'une des revendications 1 à 4, **caractérisé en ce que** l'obturation (5) a la forme d'un logement (4) pour un élément de mesure.

13. Récipient de mesure selon l'une des revendications précédentes, ayant une résistance à la pression allant jusqu'à 2 x 10⁶ P a (20 bar).
